# FASCICULE DE BREVET EUROPEEN

(11) **EP 0 700 664 B1**
(45) Date de publication et mention de la délivrance du brevet: **29.09.1999**
(21) Numéro de dépôt: 95810535.5
(22) Date de dépôt: 30.08.1995
(51) Int. Cl.: A61B 17/60, F16B 7/04

(54) **Articulation pour composants d'un fixateur externe**
Gelenkelement für äussere Fixationskomponenten
Articulation for components of an external fixator

(30) Priorité: 06.09.1994 CH 270994
(43) Date de publication de la demande: 13.03.1996
(73) Titulaire: JAQUET ORTHOPEDIE S.A., CH-1228 Plan-les-Ouates (CH)
(72) Inventeur: Mata, Jacques, CH-1163 Etoy (CH); Nyfeler, Marcel, CH-1163 Etoy (CH); Worek, Denis, CH-1255 Veyrier (CH)
(74) Mandataire: Dietlin, Henri

(56) Documents cités:
- EP-A- 0 524 441
- WO-A-88/01152
- DE-A- 1 603 999
- DE-A- 1 935 977
- US-A- 2 876 027
- US-A- 3 154 331

## Description

La présente invention est du domaine de l'orthopédie et concerne plus particulièrement une articulation pour le positionnement et la fixation des composants cylindriques - barres de fixation ou fiches osseuses - d'un fixateur externe.

De nombreux fixateurs externes connus comportent des arceaux ou cadres ayant une forme spécifique, en fonction de laquelle les fiches osseuses sont insérées dans les fragments d'os que l'on désire maintenir. On a constaté que l'on est souvent amené ainsi à insérer les fiches relativement près de nerfs ou d'artères, ce qui peut entraîner des complications.

Pour cette raison, on a développé des fixateurs externes dont les composants sont mis en place après que les fiches aient été insérées dans la position optimale par rapport au fragment osseux à maintenir et au tissu l'entourant. Les barres de fixation constituant le cadre du fixateur sont alors mises en place entre les fiches et des articulations assurent la liaison entre les barres et les fiches ou entre des barres de rigidification. Il est évidemment nécessaire que ces articulations permettent de maintenir les barres ou fiches selon une angulation variable.

Le brevet européen EP-0.321.472 (WO-88/01152) décrit une articulation pour le positionnement relatif de barres de fixation ou de fiches osseuses d'un fixateur externe. Cette articulation comporte plusieurs paires de mâchoires présentant sur leurs faces adjacentes des rainures qui forment un passage destiné à recevoir une barre ou une fiche, des moyens de blocage de la position angulaire entre lesdites paires et des moyens de serrage traversant lesdites paires de mâchoires pour serrer les barres ou fiches dans une position relative. En outre les mâchoires formant une paire sont munies respectivement d'une saillie de positionnement et d'un dégagement de forme complémentaire.

L'inconvénient d'un tel dispositif est qu'il doit être monté à l'avance sur les barres de fixation ou les fiches, qui sont introduites par leur extrémité. En variante on peut démonter ce type d'articulation avant de l'utiliser et de la remonter, en prenant garde de disposer les saillies et dégagements de positionnement vis-à-vis. En outre tant que ce dispositif n'est pas bloqué dans la position définitive voulue, les composants ne sont pas maintenus et peuvent bouger les uns par rapport aux autres et surtout l'articulation peut bouger par rapport aux barres de fixation et aux fiches sur lesquelles elle est montée de sorte que l'on est obligé de la maintenir en place avant de la serrer.

La présente invention vise à pallier ces inconvénients et a pour objet une articulation pour le positionnement relatif de barres de fixation ou de fiches d'un fixateur externe, articulation comportant plusieurs paires de mâchoires présentant sur leurs faces adjacentes des rainures qui forment un passage destiné à recevoir une barre ou une fiche, des moyens de blocage de la position angulaire entre lesdites paires et des moyens de serrage traversant lesdites paires de mâchoires pour serrer les barres ou fiches retenues entre les mâchoires dans une position relative.

Elle est caractérisée en ce que des moyens élastiques sont intercalés entre lesdites paires de mâchoires et en ce que les rainures formant ledit passage sont positionnées et agencées de manière à présenter une ouverture extérieure permettant le clipsage de la barre ou fiche par pression de cette dernière, de l'ouverture de la mâchoire dans ledit passage, à l'encontre des moyens élastiques qui pressent les faces adjacentes des mâchoires l'une contre l'autre pour maintenir l'articulation sur les barres ou fiches avant le blocage de l'articulation.

Dans une variante préférentielle, les moyens de serrage sont constitués par un arbre qui coopère avec une pièce d'arrêt pour maintenir l'empilage des mâchoires autour de l'arbre de serrage, lorsque l'on desserre celui-ci.

L'invention s'étend également à un fixateur externe comportant au moins des fiches osseuses insérées de part et d'autre de la fracture, un cadre composé de barres de fixation, et au moins une articulation disposée entre une fiche et une barre ou entre deux barres pour leur positionnement relatif. Il est caractérisé en ce que ladite articulation comporte au moins deux ouvertures extérieures permettant le clipsage de barres ou fiches par pression de celles-ci dans des passages prévus à cet effet, à l'encontre de moyens élastiques qui maintiennent les barres ou fiches entre les mâchoires constituant l'articulation avant le blocage de celle-ci.

Ce fixateur présente l'avantage d'un encombrement réduit. En outre une ou plusieurs barres peuvent être enlevées au cours de la consolidation osseuse.

Le dessin annexé représente, à titre d'exemple non limitatif, une forme d'exécution de l'objet de la présente invention.
La figure 1 est une représentation d'un raccord selon l'invention, vu en coupe dans la partie gauche et latéralement dans la partie droite du dessin.
La figure 1A est une vue agrandie d'un fragment de mâchoire, montrant une forme d'exécution des moyens de blocage de la position angulaire entre les paires de mâchoires.
La figure 2 est une vue de dessous du raccord de la figure 1.
La figure 3 est une vue en coupe transversale, selon la ligne III-III à la figure 1.
La figure 4 est une vue schématique en perspective d'un fixateur comportant différentes variantes de raccords selon l'invention, monté sur un os long.

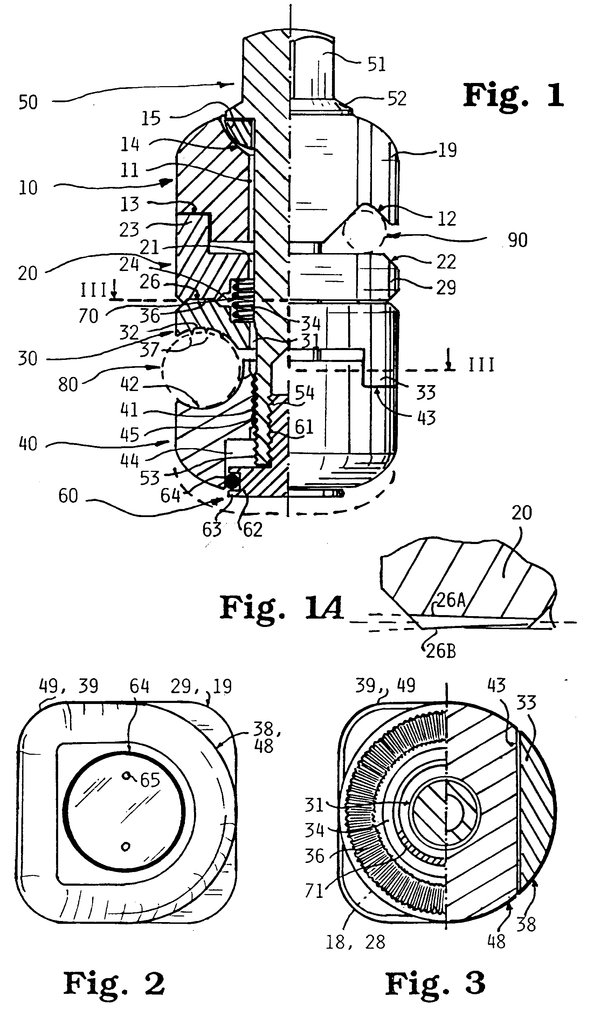

L'articulation représentée à la figure 1 est composée par l'empilage de deux paires de mâchoires constituées respectivement par une mâchoire supérieure 10 et une mâchoire intermédiaire supérieure 20, ainsi que par une mâchoire intermédiaire inférieure 30 et une mâchoire inférieure 40. Ces quatre mâchoires sont montées sur un arbre de serrage 50, qui maintient en permanence les mâchoires ensemble grâce à la pièce d'arrêt 60. Des moyens élastiques 70 sont disposés entre les mâchoires intermédiaires 20 et 30 et tendent à les écarter. On a dessiné en traitillés à la figure 1 une barre de fixation 80 et une fiche osseuse 90, vues en coupe et disposées parallèlement de part et d'autre de l'arbre 50 dans l'exemple représenté.

Dans la variante représentée à la figure 1, l'articulation est représentée dans sa position de serrage des barres ou fiches. Lorsque l'on desserre l'arbre de serrage 50, les mâchoires inférieures 30 et 40 se déplacent sous l'action des moyens élastiques jusqu'à la position basse représentée en traitillés au dessin.

La mâchoire supérieure 10 comporte une ouverture centrale 11, pour le passage de l'arbre de serrage. Sur sa face adjacente à la mâchoire intermédiaire elle comporte d'une part une rainure 12 de forme correspondant à la pièce à serrer, et d'autre part un dégagement de positionnement 13. Sur sa face opposée, la mâchoire supérieure 10 présente une creusure 14 sphérique destinée à recevoir une rondelle sphérique 15 disposée autour de la tête de l'arbre de serrage 50.

La mâchoire intermédiaire supérieure 20 présente une ouverture centrale 21. Sur sa face adjacente à la mâchoire supérieure, elle comporte un passage 22 pour la pièce à serrer et une saillie axiale 23 de forme correspondant au dégagement de positionnement 13. Sur sa face opposée, la mâchoire 20 comporte un évidement 24 pour les moyens élastiques 70 au centre de la surface 26 de contact avec l'autre mâchoire intermédiaire.

La mâchoire intermédiaire inférieure 30 comporte, de manière symétrique, une ouverture centrale 31, une rainure 32, une saillie axiale 33, un évidement central 34 et une surface de contact 36, bien visibles à la figure 3. On y notera que la surface de contact 36 peut être constituée par une couronne dentée destinée à renforcer le blocage angulaire des deux mâchoires intermédiaires. Cette denture est relativement fine pour permettre de bloquer les mâchoires intermédiaires dans un grand nombre de positions relatives.

Dans la vue agrandie de la figure 1A on notera que la surface de contact 26 est constituée par des dents radiales, dont le fond 26A est incliné de quelques degrés par rapport à l'horizontale tandis que l'arête 26B est inclinée dans le sens inverse, de manière à assurer une meilleure prise entre les mâchoires intermédiaires.

On notera encore à la figure 1 les stries 37 faisant saillie dans la rainure 32, pour assurer une meilleure prise le long de la barre 80. Il est évident que ces saillies longitudinales peuvent être remplacées par des pointes ou des matériaux évitant tout mouvement relatif des composants.

La mâchoire inférieure 40 se distingue de celles décrites jusqu'ici en ce que l'ouverture centrale 41 est taraudée de manière à recevoir les moyens de serrage 50. Cette mâchoire inférieure comporte comme la mâchoire supérieure 10 déjà décrite une rainure 42 pour la barre à positionner, un dégagement 43 coopérant avec la saillie 33 et un évidement central 44, pour la pièce d'arrêt 60. On a en outre schématisé un manchon hélicoïdal 45 de renforcement du taraudage pratiqué dans l'ouverture centrale 41 lorsque la mâchoire est réalisée en matériau léger.

Dans la variante représentée au dessin, les mâchoires superposées constituent un empilement tubulaire. On notera aux figures 2 et 3 que les mâchoires ont une forme de section circulaire 18, 28, 38 et 48 sur le côté comportant les saillies de positionnement et les dégagements de forme correspondante, tandis qu'elles ont une forme de section carrée 19, 29, 39 et 49 sur leur partie munie de rainures de réception des barres, afin d'augmenter leur surface en contact avec la pièce à positionner.

L'arbre de serrage 50 comporte à une extrémité des moyens de préhension extérieurs aux mâchoires qu'il traverse, constitué dans l'exemple donné par une tête carrée 51 évasée à sa base pour former un épaulement circulaire 52 destiné à coopérer avec la rondelle 15. A l'autre extrémité, l'arbre comporte un filetage 53 et une ouverture taraudée 54. Le filetage 53 est destiné à coopérer avec l'ouverture taraudée 41 de la mâchoire inférieure ou avec le manchon hélicoïdal 45. L'ouverture taraudée 54 reçoit la pièce d'arrêt 60.

La pièce d'arrêt 60 comporte une tige filetée 61 et un flasque de fermeture 62 dont le bord 63 comporte une gorge pour le logement d'un joint O-ring 64. Comme visible à la figure 2, le flasque 62 comporte deux trous 65 destinés à recevoir une clé de serrage lorsque l'arbre 50 est vissé dans la pièce d'arrêt 60. De préférence, ces deux pièces seront collées. La pièce d'arrêt 60 sert à maintenir l'empilage des mâchoires autour de l'arbre de serrage 50 pour éviter que ce dernier ne se désolidarise de la mâchoire inférieure.

Les moyens élastiques 70 qui tendent à écarter les mâchoires intermédiaires 20 et 30 sont plus précisément constitués par un ressort hélicoïdal de compression 70 dont les extrémités s'appuient au fond des évidements 24 et 34. La figure 1 montre la position des éléments de l'articulation quand ils sont serrés sur les pièces cylindriques 80 et 90, le ressort 70 étant comprimé entre les mâchoires intermédiaires 20 et 30 tandis que la figure 3 montre une spire 71 du ressort 70. Quand l'arbre de serrage 50 est desserré par rapport à la mâchoire inférieure 40, ce ressort 70 tend à écarter les paires de mâchoires 10,20 et 30,40, le flasque 62 venant buter au fond de l'évidement central 44. On notera encore que la hauteur des saillies 23 et 33 est supérieur à l'espace vide entre le flasque 62 et le fond de l'évidement central 44, pour assurer le positionnement des paires de mâchoires lorsque l'articulation est en position d'ouverture.

Dans la variante représentée à la figure 1, on a dessiné en traitillés une barre de fixation 80 et une fiche osseuse 90, disposées parallèlement de part et d'autre de l'arbre 50, mais dans la pratique celles-ci sont montées dans n'importe quelle relation angulaire. De manière habituelle, la barre de fixation 80 a un diamètre plus important que celui de la fiche osseuse 90. Dans ce montage, les mâchoires inférieures 30 et 40 comportent des
rainures 32, 42 rondes, dont le rayon correspond à celui de la barre de fixation 80. Les mâchoires supérieures 10 et 20 comportent respectivement une rainure 12 profilée en V et un passage 22 destiné à faciliter l'insertion latérale de la fiche osseuse. Il est également possible d'utiliser des mâchoires supérieures comportant chacune une rainure ronde lorsque l'articulation réunit deux barres de fixation, comme on le verra plus loin.

Comme on l'a déjà mentionné, le grand avantage de l'articulation décrite ici est de pouvoir être en tout temps disposée sur des fiches ou des barres qui peuvent être engagées par clipsage latéral, les mâchoires intermédiaires se déplaçant librement à l'encontre du ressort 70. Ce ressort est suffisamment puissant pour maintenir les fiches ou les barres en place, en cours de réduction et avant que le praticien ne serre l'arbre 50, ce qui permet en une seule opération de bloquer les fiches ou barres ainsi que la position angulaire des mâchoires intermédiaires.

On notera encore que lorsque l'on desserre l'arbre 50, celui-ci reste maintenu dans la mâchoire inférieure 40 en raison de la présence de la pièce d'arrêt 60, qui vient buter au fond de l'évidement 44. Le praticien ne risque donc pas de démonter involontairement les composants constituant l'articulation lorsqu'il desserre la vis 50. En variante, on peut prévoir que l'arbre de serrage traverse librement l'empilage des mâchoires pour coopérer avec une pièce d'arrêt permettant le blocage et le desserrage de l'ensemble.

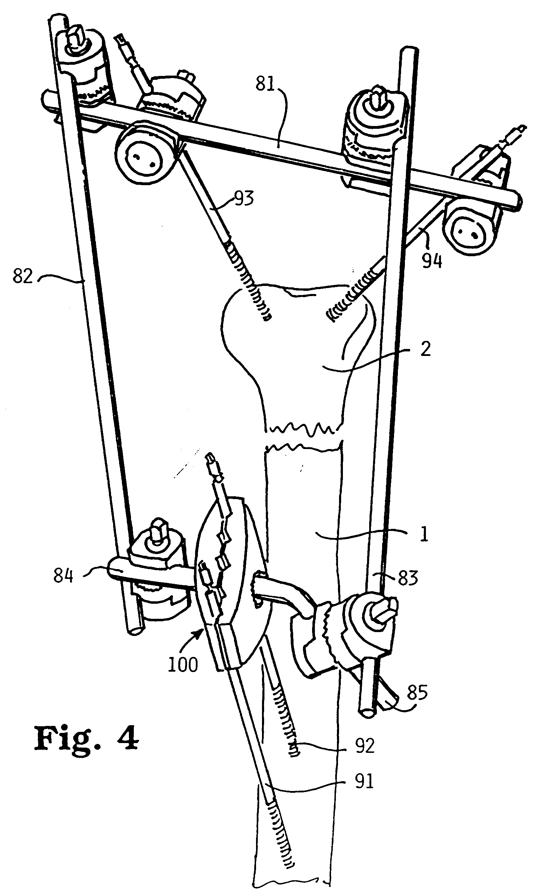

Dans l'exemple d'utilisation représenté à la figure 4, les fiches 91 et 92 sont insérées dans la diaphyse 1 d'un os fracturé aux environs de l'épiphyse 2 qui reçoit les fiches 93 et 94. Deux fiches sont représentées dans chaque fragment osseux, mais il est bien sûr possible d'en insérer davantage.

Une première barre de fixation 81 constitue la partie supérieure du cadre du fixateur, dont les barres 82 et 83 constituent les côtés. Les fiches 91 et 92 sont maintenues dans un étau 100 dont les mâchoires reçoivent deux barres 84 et 85 qui constituent la partie inférieure du cadre du fixateur externe.

Les articulations entre les fiches 93, 94 et la barre 81 sont celles représentées à la figure 1 alors que toutes les autres articulations sont disposées entre deux barres et sont par conséquent constituées par deux paires de mâchoires comportant toutes deux des rainures de section arrondie.

Il est à noter que l'on peut avantageusement employer des barres coudées, telle la barre 85 qui présente à la sortie de l'étau 100 un angle d'environ 135° ou toute autre valeur comprise entre 110° et 160° environ. Ce montage a l'avantage d'éviter la rotation de la barre dans la pièce de liaison lorsque des couples de torsion sont appliqués à l'ensemble en améliorant la décomposition des forces. De plus il permet de disposer le fixateur aussi près que possible du membre fracturé pour diminuer l'encombrement de l'ensemble.

Pour réduire le poids du fixateur, on choisira de réaliser certaines pièces en alliage léger tel qu'aluminium ou tout matériau composite, par exemple pour les mâchoires extérieures 10 et 40. Quant aux mâchoires intermédiaires 20 et 30, elles seront de préférence en acier, afin que les dentures 26 et 36, ainsi que les stries 37 ne se déforment pas.

## Revendications

1. Articulation pour le positionnement relatif de barres de fixation (80,81,82,83,84,85) ou de fiches (90,91,92,93,94) d'un fixateur externe, articulation comportant plusieurs paires de mâchoires (10,20; 30,40) présentant sur leurs faces adjacentes des rainures (12,22,32,42) qui forment un passage destiné à recevoir une barre ou une fiche, des moyens de blocage de la position angulaire entre lesdites paires et des moyens de serrage (50) traversant lesdites paires de mâchoires pour serrer les barres ou fiches retenues entre les mâchoires dans une position relative, caractérisée en ce que des moyens élastiques (70) sont intercalés entre lesdites paires de mâchoires (10,20; 30,40) et en ce que les rainures (12,22,32,42) formant ledit passage sont positionnées et agencées de manière à présenter une ouverture extérieure permettant le clipsage de la barre (80) ou fiche (90) par pression de cette dernière, de l'ouverture de la mâchoire dans ledit passage, à l'encontre des moyens élastiques qui pressent les faces adjacentes des mâchoires l'une contre l'autre pour maintenir l'articulation sur les barres ou fiches avant le blocage de l'articulation.

2. Articulation selon la revendication 1, caractérisée en ce que lesdits moyens élastiques (70) sont constitués par un ressort hélicoïdal disposé autour d'un arbre central (50) constituant lesdits moyens de serrage.

3. Articulation selon la revendication 2, caractérisée en ce qu'elle comprend une pièce d'arrêt 60 coopérant avec ledit arbre central et apte à maintenir l'empilage des mâchoires (10,20,30,40) sur ledit arbre.

4. Articulation selon la revendication 1, caractérisée en ce que les mâchoires sont de forme générale arrondie et présentent des épaulement (19,29,39,49) de forme carrée dans leur partie munie des rainures (12,22, 32,42).

5. Articulation selon la revendication 1, caractérisée en ce que lesdites rainures sont de forme arrondie.

6. Articulation selon la revendication 5, caractérisée en ce que lesdites rainures comportent des saillies 37 destinées à améliorer le blocage des barres.

7. Articulation selon la revendication 1, caractérisée en ce que lesdites rainures sont constituées par au moins deux surfaces planes sécantes.

8. Articulation selon la revendication 1, caractérisée en ce que les moyens de blocage de la position angulaire entre lesdites paires sont constitués par des surfaces de contact (26,36) comportant des dentures radiales dont le fond (26A) est incliné de quelques degrés par rapport à un plan perpendiculaire à l'arbre (50) de serrage tandis que l'arête (26B) est inclinée dans le sens inverse, de manière à assurer une meilleure prise entre les mâchoires intermédiaires.

9. Fixateur externe comportant au moins :
- des fiches osseuses (90,91,92,93,94) insérées de part et d'autre de la fracture,
- un cadre composé de barres de fixation (80,81,82,83,84,85),
- au moins une articulation disposée entre une fiche et une barre ou entre deux barres pour leur positionnement relatif,
caractérisée en ce que ladite articulation comporte au moins deux ouvertures extérieures permettant le clipsage de barres (80) ou fiches (90) par pression de celles-ci dans des passages prévus à cet effet, à l'encontre de moyens élastiques (70) qui maintiennent les barres ou fiches entre les mâchoires constituant l'articulation avant le blocage de celle-ci.

10. Fixateur externe selon la revendication 9, caractérisé par au moins une barre (85) présentant sur sa longueur un angle compris entre 110° et 160°, apte à éviter tout glissement de la barre dans l'articulation lorsqu'elle est soumise à torsion et à réduire l'encombrement du fixateur.

## Claims

1. Articulation for the relative positioning of fixation bars (80,81,82,83,84,85) or of pins (90,91,92,93,94) of an external fixator, which articulation includes a plurality of pairs of jaws (10,20; 30,40) having on their adjacent faces grooves (12,22,32,42) which form a passage intended to receive a bar or a pin, means for locking the relative angular position of the said pairs and clamping means (50) passing through the said pairs of jaws, for clamping the bars or pins held between the jaws in a relative position, characterized in that elastic means (70) are interposed between the said pairs of jaws (10,20; 30,40) and in that the grooves (12,22,32,42) forming the said passage are positioned and arranged so as to have an external opening allowing the bar (80) or pin (90) to be clipped in by pressure on it from the opening of the jaw into the said passage, against the elastic means which press the adjacent faces of the jaws against one another in order to hold the articulation on the bars or pins before locking of the articulation.

2. Articulation according to Claim 1, characterized in that the said elastic means (70) consist of a coil spring arranged around a central shaft (50) constituting the said clamping means.

3. Articulation according to Claim 2, characterized in that it comprises a stop piece (60) interacting with the said central shaft and capable of holding the stack of jaws (10,20,30,40) on the said shaft.

4. Articulation according to Claim 1, characterized in that the jaws are of rounded general shape and have shoulders (19,29,39,49) of square shape in their part which is provided with grooves (12.22.32.42).

5. Articulation according to Claim 1, characterized in that the said grooves are of rounded shape.

6. Articulation according to Claim 5, characterized in that the said grooves have projections (37) intended to improve locking of the bars.

7. Articulation according to Claim 1, characterized in that the said grooves consist of at least two secant plane surfaces.

8. Articulation according to Claim 1, characterized in that the means for locking the relative angular position of the said pairs consists of contact surfaces (26,36) having radial teeth, of which the bottom (26A) is inclined by a few degrees with respect to a plane perpendicular to the clamping shaft (50), whereas the edge (26B) is inclined in the opposite direction, so as to ensure better engagement between the intermediate jaws.

9. External fixator including at least:
- osseous pins (90,91,92,93,94) inserted on either side of the fracture,
- a frame composed of fixation bars (81,81,82,83,84,85),
- at least one articulation arranged between a pin and a bar or between two bars for their relative positioning,
characterized in that the said articulation includes at least two external openings allowing bars (80) or pins (90) to be clipped in by pressure on them into passages provided for this purpose, against elastic means (70) which hold the bars or pins between the jaws constituting the articulation before it is locked.

10. External fixator according to Claim 9, characterized by at least one bar (85) having, along its length, an angle lying between 110° and 160°, capable of preventing any sliding of the bar in the articulation when it is subjected to torsion and or reducing the bulk of the fixator.

## Patentansprüche

1. Gelenkverbindung zum relativen Positionieren von Befestigungsstangen (80,81,82,83,84,85) oder Steckverbinder (90,91,92,93,94) eines äusseren Fixateurs, welche mehrere Paare von Klemmbacken (10,20; 30,40) aufweist, die in ihren anliegenden Flächen Einkerbungen (12,22,32,42) enthalten, einen Durchgang für eine Befestigungsstange oder einen Steckverbinder bilden und Mittel zum Blockieren (50) der Winkelstellung zwischen den Klemmbacken und Spannmittlen, welche diese Klemmbacken durchqueren, umfassen, um auf diese Weise die zwischen den Klemmbacken festgehaltenen Befestigungstangen oder Steckverbinder in einer relativen Stellung zueinander einzuspannen, dadurch gekennzeichnet, dass zwischen den Paaren von Klemmbacken (10,20;30,40) elastiche Mittel (70) angeordnet sind und dass die den Durchgang bildenden Einkerbungen (12,22,32,42) so positioniert und ausgelegt sind, dass sie eine äussere Oeffnung bilden, welche das Einstecken einer Befestigungsstange (80) oder eines Steckverbinders (90) in den Durchgang durch Ausüben von Druck auf die Klemmbacken und Auseinanderdrücken derselben gegen die Wirkung der elastischen Mittel ermöglicht, so dass die Gelenkverbindung an den Befestigungsstangen oder den Steckverbindern festgehalten wird, bevor die Gelenkverbindung blockiert wird.

2. Gelenkverbindung nach Anspruch 1, dadurch gekennzeichnet, dass die elastischen Mittel (70) eine Schraubenfeder sind, welche um eine zentrale Welle (50) angeordnet ist, die das Spannmittel bildet.

3. Gelenkverbindung nach Anspruch 2, dadurch gekennzeichnet, dass sie eine Arretierung (60) aufweist, welche mit der zentralen Welle zusammenwirkt und geeignet ist, die lagenweise Anordnung der Klemmbacken (10,20,30,40) auf der Welle zu halten. zentralen Welle zusammenwirkt und geeignet ist, die lagenweise Anordnung der Klemmbacken (10,20,30,40) auf der Welle zu halten.

4. Gelenkverbindung nach Anspruch 1, dadurch gekennzeichnet, dass die Klemmbacken eine weitgehend abgerundete Form haben und in ihrem mit den Einkerbungen (12,22,32,42) versehenen Teil viereckige Schultern (19,29,39,49) aufweisen.

5. Gelenkverbindung nach Anspruch 1, dadurch gekennzeichnet, dass die Einkerbungen eine abgerundete Form haben.

6. Gelenkverbindung nach Anspruch 5, dadurch gekennzeichnet, dass die Einkerbungen Nocken (37) aufweisen, welche dafür bestimmt sind, die Blockierung der Befestigungsstangen zu verbessern.

7. Gelenkverbindung nach Anspruch 1, dadurch gekennzeichnet, dass die Einkerbungen durch mindestens zwei sich schneidende ebene Flächen gebildet sind.

8. Gelenkverbindung nach Anspruch 1, dadurch gekennzeichnet, dass die Mittel zum Blockieren der Winkeleinstellung zwischen den Paaren von Klemmbacken Kontaktflächen (26,36) sind, welche radiale Verzahnungen aufweisen, deren Grund (26A) um einige Grade gegenüber der rechtwinkligen Ebene der Spannwelle (50) geneigt ist, um so einen besseren Eingriff zwischen den intermediären Klemmbacken zu gewährleisten.

9. Aeusserer Fixateur, welcher mindestens:
- Steckverbinder für Knochen (90,91,92,93,94), welche auf beiden Seiten der Bruchstelle in Knochen eingeführt werden,
- einem aus Befestigungsstangen (80,81,82,83,84,85) gebildeten Rahmen,
- mindestens eine Gelenkverbindung, welche zwischen einem Steckverbinder und einer Befestigungsstange oder zwischen zwei Befestigungsstangen für ihre relative Positionierung angeordnet sind,
aufweist, dadurch gekennzeichnet, dass die Gelenkverbindungen mindestens zwei äussere Oeffnungen aufweist, die das Einklemmen von Befestigungsstangen (80) oder Steckverbindern (90) durch Ausübung von Druck in für diesen Zweck vorgesehene Druchgänge entgegen der Wirkung von elastischen Mitteln (70) erlauben, welche die Befestigungsstangen oder Steckverbinder zwischen den Klemmbacken, welche die Gelenkverbindung bilden, festhalten, bevor diese Gelenkverbindung blockiert wird.

10. Aeusserer Fixateur nach Anspruch 9, dadurch gekennzeichnet, dass er mindestens eine abgebogene Befestigungsstange (85) umfasst, deren Abschnitte einen Winkel zwischen 110° und 160° zueinander aufweisen, um so ein Gleiten der Befestigungsstange in der Gelenkverbindung zu verhindern, wenn diese Torsionskräften unterworfen wird, und um auf diese Weise die Abmessungen des Fixateurs zu reduzieren.
